(19) Europäisches Patentamt
European Patent Office
Office européen des brevets

(11) **EP 3 581 106 B1**

(12) **EUROPEAN PATENT SPECIFICATION**

(45) Date of publication and mention
of the grant of the patent:
**14.12.2022 Bulletin 2022/50**

(21) Application number: **18751149.8**

(22) Date of filing: **06.02.2018**

(51) International Patent Classification (IPC):
*A61B 5/11* (2006.01)      *A61B 5/00* (2006.01)
*A61B 5/08* (2006.01)      *A61B 5/113* (2006.01)
*A61B 5/0205* (2006.01)

(52) Cooperative Patent Classification (CPC):
**A61B 5/0816; A61B 5/00; A61B 5/0205;**
**A61B 5/11; A61B 5/113; A61B 5/6891;**
A61B 5/0077; A61B 5/01; A61B 5/02141;
A61B 5/02444; A61B 5/029; A61B 5/091;
A61B 5/1115; A61B 5/1117; A61B 5/4809;   (Cont.)

(86) International application number:
**PCT/JP2018/003929**

(87) International publication number:
**WO 2018/147252 (16.08.2018 Gazette 2018/33)**

(54) **BED MONITORING SYSTEM**

BETTÜBERWACHUNGSSYSTEM

SYSTÈME DE SURVEILLANCE DE LIT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **10.02.2017   JP 2017023032**

(43) Date of publication of application:
**18.12.2019   Bulletin 2019/51**

(73) Proprietors:
• **Minebea Mitsumi Inc.**
  **Kitasaku-gun, Nagano 389-0293 (JP)**
• **National University Corporation Chiba University**
  **Chiba-shi, Chiba 263-8522 (JP)**

(72) Inventors:
• **AKATSU, Hiroyuki**
  **Kitasaku-gun**
  **Nagano 389-0293 (JP)**
• **IIDA, Norihito**
  **Kitasaku-gun**
  **Nagano 389-0293 (JP)**
• **ISONO, Shiroh**
  **Chiba-shi**
  **Chiba 260-8670 (JP)**

(74) Representative: **Novagraaf Group**
**Chemin de l'Echo 3**
**1213 Onex (CH)**

(56) References cited:
**WO-A1-2017/018506      WO-A1-2017/056476**
**WO-A2-2011/009085      JP-A- 2016 144 627**
**US-A1- 2015 022 343**

**(Cont. next page)**

(52) Cooperative Patent Classification (CPC): (Cont.)
A61B 5/4818

**Description**

TECHNICAL FIELD

The present invention relates to a bed monitoring system configured to monitor conditions of a subject on a bed by using load detectors and other information detecting units.

BACKGROUND ART

There are proposed methods of using load detector and the like to noninvasively monitor conditions of a patient or a care receiver on a bed, in the sites of medical practice and care service.
WO 2011/009085 A1 describes a bed monitoring system which calculates a center of pressure and determines respiration, heart rate and movement from a load cell.
Patent Literature 1 discloses a present-on-bed detecting method for determining whether or not a subject is present on a bed by detecting whether or not the subject on the bed has respirations, on the basis of the outputs of a plurality of load detectors arranged under the legs of the bed.
Patent Literature 2 discloses a biological and physiological detecting device configured to detect the respiratory rate, pulse rate, presence of snore and the like of a subject on a bed, based on the outputs from a plurality of load detecting sensors arranged under the legs of the bed.

**Citation List**

Patent Literature 1: Japanese Patent Application Laid-open No. 2008-264338
Patent Literature 2: Japanese Patent No. 4883380

SUMMARY

**Technical Problem**

With such noninvasive monitoring as disclosed in Patent Literatures 1 and 2, it is desired to improve the monitoring precision. Further, items which are monitorable by the method shown in Patent Literature 1 or with the device shown in Patent Literature 2 are limited, and thus, it is difficult to say that the needs in the sites of medical practice and/or care service are sufficiently satisfied.
In view of the above, an object of the present invention is to provide a bed monitoring system capable of noninvasively and precisely monitoring various items about a subject.

**Solution to the Problem**

According to the present invention, there is provided a bed monitoring system for monitoring a subject on a bed, as defined in claim
In the bed monitoring system according to the first aspect, the body motion information may include an information on a large body motion of the subject and an information on a small body motion of the subject, an amount of movement of the center of gravity position of the subject within a predetermined time period caused by the small body motion being smaller than an amount of movement of the center of gravity position of the subject within the predetermined time period caused by the large body motion, and
the body motion information determining unit may include a first body motion information determining unit configured to determine the information on the large body motion of the subject and a second body motion information determining unit configured to determine the information on the small body motion of the subject.
In the bed monitoring system according to the first aspect, the subject information detecting unit may include an image information detecting unit configured to detect the image information of the subject, an audio information detecting unit configured to detect the audio information of the subject, and a temperature information detecting unit configured to detect the temperature information of the subject.
According to a second aspect of the present invention, there is provided a bed system including:

a bed; and
the bed monitoring system according to the first aspect.

**EFFECT of the INVENTION**

According to the bed monitoring system of the present invention, it is possible to noninvasively and precisely monitor various items about the subject.

BRIEF DESCRIPTION OF THE DRAWINGS

Fig. 1 is a block diagram depicting a configuration of a bed monitoring system according to an embodiment of the present invention.
Figs. 2(a) and 2(b) are illustrative views for explaining an arrangement of a bed, load detectors, an image information detecting unit, an audio information detecting unit, and a temperature information detecting unit. Fig. 2(a) depicts the arrangement of the load detectors with respect to the bed. Fig. 2(b) depicts the arrangement of the image information detecting unit, the audio information detecting unit and the temperature information detecting unit with respect to the bed.
Fig. 3 is a flow chart depicting a monitoring method according to the embodiment of the present invention.
Fig. 4 is a block diagram depicting a configuration of a body motion information estimating unit.
Fig. 5(a) depicts an example of center of gravity locus of a subject.
Fig. 5(b) depicts a center of gravity locus obtained by converting the center of gravity locus depicted in Fig. 5(a) into a locus based on a low sampling frequency.
Figs. 6(a), 6(b) and 6(c) depict loci where large body motion loci are removed from the center of gravity locus of the subject on a bed depicted in Fig. 5(a).
Fig. 7 is an illustrative view for depicting a case of decomposing the center of gravity locus into a component forming a small body motion locus and a component forming a respiratory oscillation locus.
Figs. 8(a), 8(b) and 8(c) depict loci where small body motion loci are removed from the loci depicted in Figs. 6(a), 6(b) and 6(c), respectively.
Fig. 9 depicts an example of respiratory oscillation locus which is rotated to let its oscillation direction conform to an X axis direction.
Fig. 10 is an illustrative view for explaining a method for determining a movement direction of a motion vector.
Fig. 11 depicts a case of decomposing the center of gravity locus including the small body motion locus and the respiratory oscillation locus of the subject, into a plurality of motion vectors.
Fig. 12 is a block diagram depicting an overall configuration of the bed system according to the embodiment of the present invention.

EMBODIMENTS

<Embodiment>

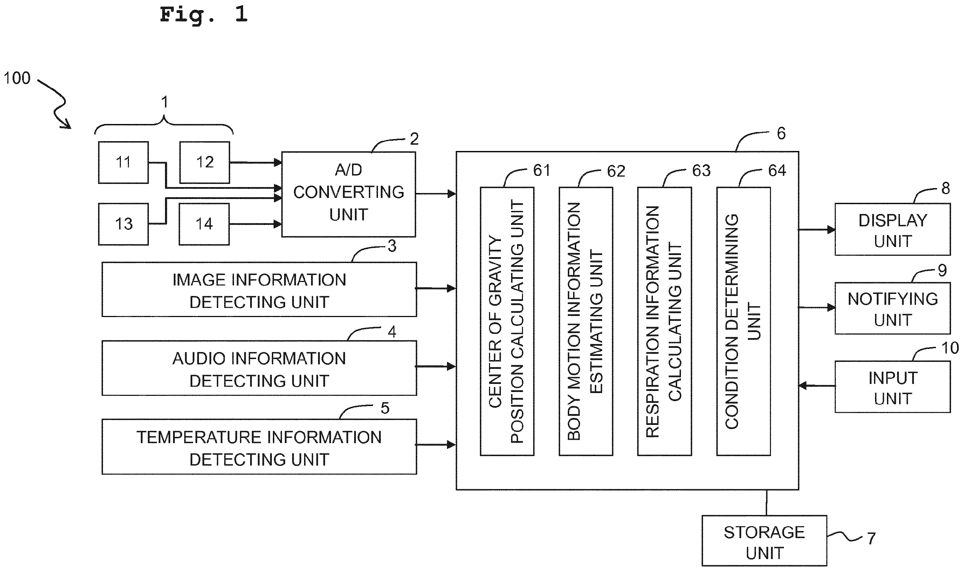

An embodiment of the present invention will be explained with reference to Figs. 1 to 11.

As depicted in Fig. 1, a bed monitoring system 100 of this embodiment has, chiefly, a load detecting unit 1, an image information detecting unit 3, an audio information detecting unit 4, a temperature (thermal) information detecting unit 5, a control unit 6, a storage unit 7, and a display unit 8. The load detecting unit 1 and the control unit 6 are connected via an A/D converting unit 2. The control unit 6 is further connected with a notifying unit 9 and an input unit 10.

The load detecting unit 1 is provided with four load detectors 11, 12, 13, 14. Each of the load detectors 11, 12, 13, 14 is a load detector which detects an item of load by using, for example, a beam-type load cell. Such kind of load detectors are described in, for example, Japanese Patent No. 4829020 and Japanese Patent No. 4002905. Each of the load detectors 11, 12, 13, 14 is connected to the A/D converting unit 2 by means of wiring.

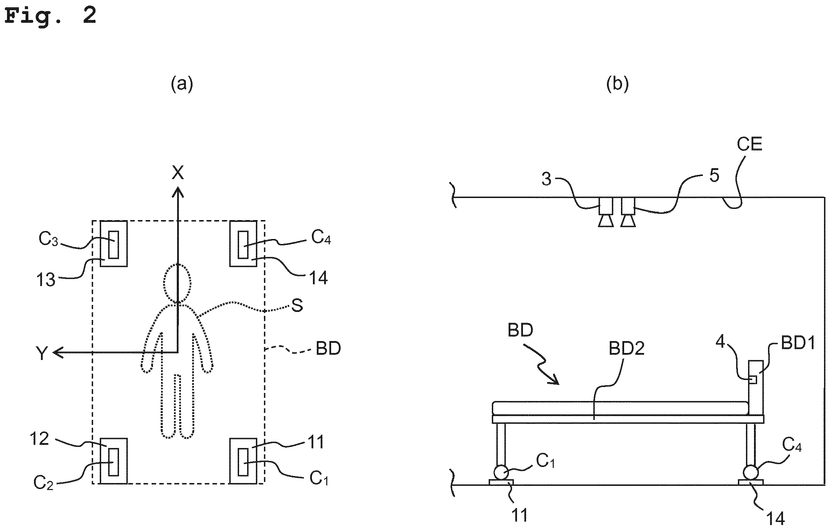

The four load detectors 11, 12, 13, 14 of the load detecting unit 1 are arranged under the legs of a bed to be used by a subject (human subject). In particular, as depicted in Figs. 2(a) and 2(b), the load detectors 11, 12, 13, 14 are arranged respectively under casters $C_1$, $C_2$, $C_3$, $C_4$ attached to lower end portions of the legs disposed at the four corners of the bed BD.

The A/D converting unit 2 is provided with an A/D converter which converts an analog signal fed from the load detecting unit 1 into a digital signal. The A/D converting unit 2 is connected to each of the load detecting unit 1 and the control unit 6 by means of wiring.

The image information detecting unit 3 is, in this embodiment, a video camera which is fitted on a ceiling CE above the bed BD so as to be capable of shooting (taping, photographing) the entire area of the upper surface (upside) of the bed BD. The image information detecting unit 3 is provided with an infrared shooting function such that the subject on the bed BD can be shot even in the dark.

The audio information detecting unit 4 is, in this embodiment, a slim microphone provided on a headboard BD1 of the bed BD. The audio information detecting unit 4 is imbedded (built-in) in the headboard BD1 positioned close to the head of the subject such that the respiratory sound and the like of the subject on the bed BD can be well collected.

The temperature information detecting unit 5 is, in this embodiment, an infrared thermography device fitted on the ceiling CE above the bed BD to be capable of detecting the entire area of the upper surface of the bed BD.

Each of the image information detecting unit 3, the audio information detecting unit 4, and the temperature information detecting unit 5 is connected to the control unit 6 by means of wiring.

The control unit 6 is an exclusive or general-purpose computer. A center of gravity position calculating unit 61, a body motion information estimating unit (body motion information determining unit) 62, a respiration information calculating unit 63, and a condition determining unit 64 are constructed in the control unit 6.

The storage unit 7 is a storage device which stores various items of detected/estimated/calculated information, and referential data and the like used for determining conditions of the subject, in the bed monitoring system 100. For example, it is possible to use a hard disk (magnetic disk) therefor. The display unit 8 is a monitor such as a liquid crystal monitor or the like for displaying the information outputted from the control unit 6 for a user of the bed monitoring system 100.

The notifying unit 9 is provided with a device such as a speaker, for example, for auditorily performing predetermined notifications on the basis of the information fed from the control unit 6. The input unit 10 is an interface for performing predetermined inputs for the control unit 6, which may be a keyboard and a mouse.

An explanation will be made about the operation for monitoring the subject on the bed, by using the bed monitoring system 100 as described above. Here, as an example, monitoring the subject includes finding an appearance (aspect) of a body motion and/or respiration of the subject, determining whether or not the subject is present on the bed BD, determining whether the subject is alive or dead, and the like (details will be described later).

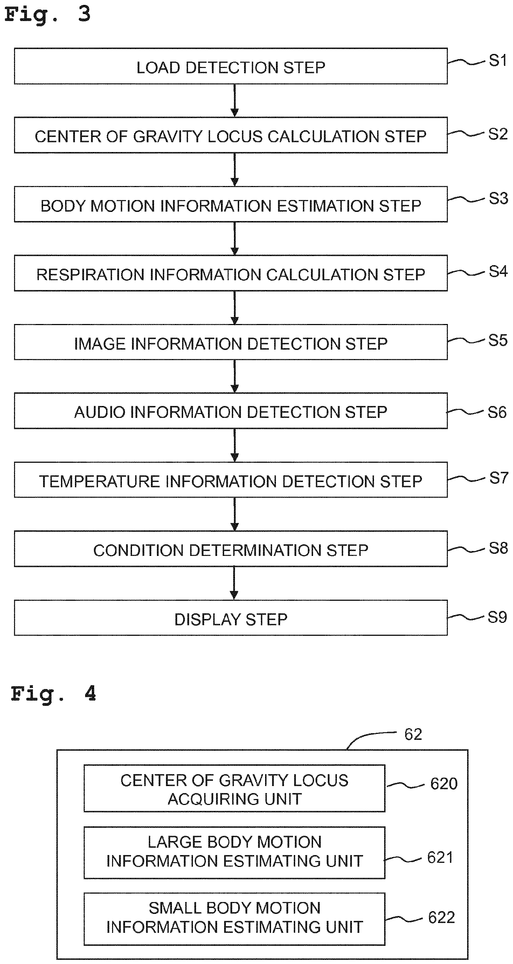

As depicted in Fig. 3, monitoring the subject by using the bed monitoring system 100 includes a load detection step (S1) for detecting a load of the subject, a center of gravity locus calculation step (S2) for calculating a temporal variation of the center of gravity position (the center of gravity locus) of the subject on the basis of the detected load, a body motion information estimation step (S3) for estimating (determining) information about a body motion of the subject on the basis of the found (obtained) center of gravity locus, a respiration information calculation step (S4) for calculating information about respirations of the subject on the basis of the found (obtained) center of gravity locus, an image information detection step (S5) for detecting image information about the subject on the bed, an audio information detection step (S6) for detecting audio information about the subject on the bed, a temperature information detection step (S7) for detecting temperature information about the subject on the bed, a condition determination step (S8) for determining conditions of the subject on the basis of the center of gravity position, body motion information, respiration information, image information, audio information and temperature information of the subject, and a display step (S9) for displaying the determined conditions of the subject.

<Load Detection Step>

In the load detection step S1, the load detectors 11, 12, 13, 14 are used to detect the load of the subject (human subject) S on the bed BD. Because the load detectors 11, 12, 13, 14 are arranged respectively under casters $C_1$, $C_2$, $C_3$, $C_4$ as described above, the load, which is applied to the upper surface of the bed BD, is detected in a dispersed manner by the four load detectors 11, 12, 13, 14.

Each of the load detectors 11, 12, 13, 14 detects the load (load change), and the load (load change) is outputted as analog signals to the A/D converting unit 2. The A/D converting unit 2 converts the analog signals into digital signals while using the sampling period of, for example, 5 milliseconds, and the digital signals (referred to hereinafter as "load signals") is outputted to the control unit 6. Hereinafter, the load signals outputted from load detectors 11, 12, 13, 14, and converted into digital signals by the A/D converting unit 2 are referred to as load signals $s_1$, $s_2$, $s_3$, $s_4$, respectively.

<Center of Gravity Locus Calculation Step>

In the center of gravity locus calculation step S2, the center of gravity position calculating unit 61 calculates the position G (X, Y) of the center of gravity G of the subject S on the bed BD at a predetermined period T (for example, a period equal to the sampling period of 5 milliseconds described above) on the basis of the load signals si to $s_4$ fed from the load detectors 11 to 14, to obtain (find) the temporal variation of the position of the center of gravity G (the center of gravity locus GT) of the subject S. In this case, (X, Y) indicates the coordinates on the XY coordinate plane in which X extends in the longitudinal (lengthwise) direction of the bed BD and Y extends in the lateral (widthwise) direction of the bed BD while the central portion of the bed BD is the origin (Fig. 2(a)).

The calculation of the position G (X, Y) of the center of gravity G by the center of gravity position calculating unit 61 is performed in accordance with the following operation. That is, G (X, Y) is calculated in accordance with the following formulas assuming that the coordinates of the load detectors 11, 12, 13, 14 are $(X_{11}, Y_{11})$, $(X_{12}, Y_{12})$, $(X_{13}, Y_{13})$, and $(X_{14}, Y_{14})$ respectively, and the detected values (load values) of the load detectors 11, 12, 13, 14 are $W_{11}$, $W_{12}$, $W_{13}$, and $W_{14}$ respectively.

Formula 1:

$$X = \frac{X_{11} \times W_{11} + X_{12} \times W_{12} + X_{13} \times W_{13} + X_{14} \times W_{14}}{W_{11} + W_{12} + W_{13} + W_{14}}$$

Formula 2:

$$Y = \frac{Y_{11} \times W_{11} + Y_{12} \times W_{12} + Y_{13} \times W_{13} + Y_{14} \times W_{14}}{W_{11} + W_{12} + W_{13} + W_{14}}$$

The center of gravity position calculating unit 61 calculates the position G (X, Y) of the center of gravity G at the predetermined period T on the basis of the above Formula 1 and Formula 2 while obtaining the temporal variation of the position G (X, Y) of the center of gravity G, that is, the center of gravity locus GT, and then causes the storage unit 7 to store the obtained center of gravity locus GT.

<The body motion information estimation step S3 and the respiration information calculation step S4>

In the body motion information estimation step S3, the body motion information estimating unit 62 estimates the information about the body motion of the subject on the basis of the center of gravity locus GT calculated in the center of gravity locus calculation step S2. In the respiration information calculation step S4, the respiration information calculating unit 63 calculates the information about the respirations of the subject (a respiratory rate, a respiratory ventilation volume (tidal volume), and the like) on the basis of the center of gravity locus GT calculated in the center of gravity locus calculation step S2, and the information about the body motion of the subject.

The information about the body motion of the subject S is estimated, and the information about the respiration of the subject S is calculated, on the basis of the following principle.

The inventors of the present invention have found out, on the basis of observation of the biological activities (including the body movement and respiration) of the subject S on the bed BD and observation of aspects of the temporal variation of the position of the center of gravity G of the subject, that it is possible to classify the biological activities of the subject S into "large body motion", "small body motion" and "respiration"; and that the locus of temporal variation of the position of center of gravity G according to a large body motion (to be referred to below as "large body motion locus"), the locus of temporal variation of the position of center of gravity G according to a small body motion (to be referred to below as "small body motion locus"), and the locus of temporal variation of the position of center of gravity G according to the respiration (to be referred to below as "respiratory oscillation (vibration) locus") have features different from each other.

In this specification and in the present invention, the "large body motion" refers to a comparatively large motion among the body motions of the subject, along with a torso (body-trunk) motion such as, in particular, a turn-over, get-up or the like. If the subject performs a large body motion, then generally speaking, the direction of the body axis of the subject (the direction in which the backbone of the subject extends) will be changed.

If the large body motion is defined in terms of an aspect of a temporal variation of the position of the center of gravity G, then generally it is possible to define the large body motion as a movement of the center of gravity G through a comparatively long distance which is longer than a predetermined distance within a predetermined period, that is, a body motion which causes the move of the center of gravity G at a comparatively high speed. In particular, for example, it is possible to define the large body motion as a body motion which causes the movement of the center of gravity at a speed exceeding a predetermined value V. Alternatively, on the basis of the difference from the temporal variation of the position of center of gravity G arising from the small body motion, for example, it is also possible to define the large body motion as a body motion to move the center of gravity G through a distance longer than a predetermined multiple of the distance of the movement of the center of gravity G due to the small body motion in a unit of time. The large body motion locus is a locus of such a movement of the center of gravity G.

In this specification and in the present invention, the "small body motion" refers to a comparatively small motion among the body motions of the subject, without the torso (body-trunk) motion such as, in particular, the mere motion of a hand, a foot and/or the head, and the like.

If the small body motion is defined in terms of an aspect of a temporal variation of the position of the center of gravity G, then generally it is possible to define the small body motion as a movement of the center of gravity G through a comparatively short distance in a unit of time, that is, a body motion which causes the move of the center of gravity G at a comparatively low speed. In particular, for example, it is possible to define the small body motion as a body motion where the movement of the center of gravity occurs at a speed taking the predetermined value v or so.

Further, depending on the contents determined in the condition determination step S8, a body motion as described below may be defined as the small body motion. That is, the body motion which causes the center of gravity movement having the movement speed of the predetermined value v or so, having no periodicity, and moving in a direction different from a body axis direction of the subject S. For example, it is possible to adopt such a definition if spasms of the hands and feet are excludable from the "small body motion" in determining the physical condition (body condition). The small body motion locus is a locus of such a movement of the center of gravity G.

An aspect of the temporal variation of the position of the center of gravity G according to respiration is described as follows. The human respiration is performed by moving the chest and the diaphragm to expand and shrink the lungs. In this context, when the air is inhaled, i.e., when the lungs are expanded, the diaphragm is lowered downwardly, and the internal organs are also moved downwardly. On the other hand, when the air is expired, i.e., when the lungs are shrunk, the diaphragm is raised upwardly, and the internal organs are also moved upwardly. As a result of the research on such kind of respiration performed by the inventors of the present invention, it has been found out that because the respiration accompanies such up/down motion of the internal organs, the center of gravity G oscillates due to the respiration, approximately along the up/down direction of the subject (the direction along the backbone), i.e., the body axis direction. Therefore, it is understood that it is possible to distinguish the respiration present as the oscillation of the center of gravity in the body axis direction from the small body motion present as the movement of the center of gravity in a different direction from the body axis direction.

The body motion information estimating unit 62 of this embodiment specifies the large body motion locus and the small body motion locus included in the locus of the movement of center of gravity of the subject on the basis of the fact that the large body motion locus, the small body motion locus, and the respiratory oscillation locus have the different features as described earlier on. The body motion information estimating unit 62 estimates the contents of the body motion of the subject on the basis of the specified large body motion locus and small body motion locus.

In the same manner, the respiration information calculating unit 63 of this embodiment specifies the respiratory oscillation locus included in the locus of the movement of center of gravity of the subject, and calculates the respiration information of the subject such as the respiratory rate and the respiratory ventilation volume on the basis of the specified respiratory oscillation locus.

A particular example will be described as follows for the body motion information estimating unit 62 to estimate the body motion information of the subject S on the basis of the center of gravity locus GT of the subject S.

As shown in Fig. 4, the body motion information estimating unit 62 includes a center of gravity locus acquiring unit 620 for extracting the center of gravity locus of the subject S from the storage unit 7, a large body motion information estimating unit 621 (first body motion information determining unit) for estimating an appearance (aspect) of the large body motion of the subject S on the basis of the extracted center of gravity locus GT, and a small body motion information estimating unit 622 (second body motion information determining unit) for estimating an appearance of the small body motion of the subject S on the basis of the extracted center of gravity locus GT.

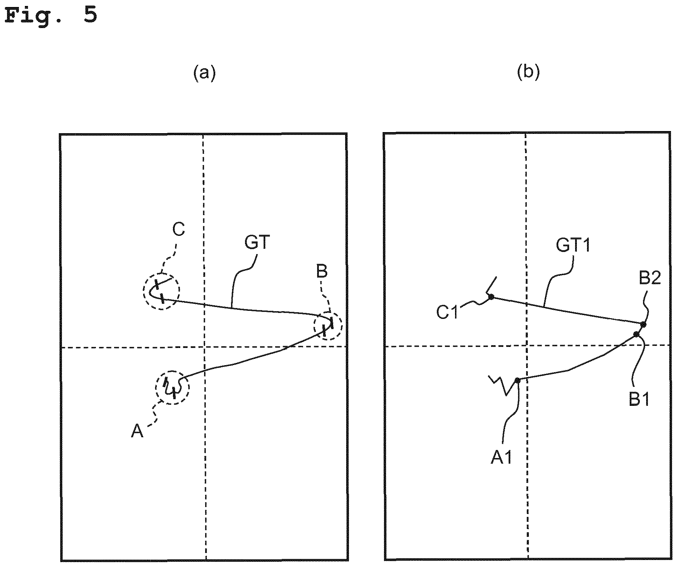

The body motion information estimating unit 62 first uses the center of gravity locus acquiring unit 620 to extract from the storage unit 7 the center of gravity locus GT of the subject S during a predetermined period. An example of the extracted center of gravity locus GT is depicted in Fig. 5(a). The center of gravity locus GT depicted in Fig. 5(a) indicates the fact that the subject S makes one reciprocating motion in the left-right direction on the bed in accordance with the large body motion (turning over or the like). Further, the center of gravity locus GT indicates that the center of gravity G of the subject S moves in each of the areas A, B, C during the period in which no large body motion occurs (hereinafter, referred to as "stable posture period"). The movement of the center of gravity G in the areas A, B, C is caused by the respiration and the small body motion of the subject S.

Next, the body motion information estimating unit 62 specifies the large body motion locus of the subject S from the center of gravity locus GT by using the large body motion information estimating unit 621. The large body motion information estimating unit 621 analyzes the position of the center of gravity G at each sampling time, and can properly specify the locus of the movement of the center of gravity G according to a large body motion (the large body motion locus) on the basis of the definition of the "large body motion". In particular, for example, if the center of gravity G moves beyond a predetermined distance within a predetermined time, then the locus of its movement is specified as the large body motion locus.

The large body motion information estimating unit 621 uses the following method to determine whether or not the center of gravity G moves beyond the predetermined distance within the predetermined time. At first, the center of gravity locus GT depicted in Fig. 5(a) is converted into a center of gravity locus GT1 based on a lower sampling frequency (Fig. 5(b)). The conversion can be performed by thinning out the data of the center of gravity position G acquired at a sampling frequency of 5 millisecond and/or by using the moving average process. Alternatively, the conversion can be also performed by subjecting the center of gravity locus GT to the frequency resolution and extracting the predetermined low frequency component by means of a low-pass filter. Note that it is desirable that the low sampling frequency has the period which is short (the frequency which is large) to such an extent that the large body motion is sufficiently extracted, and the low sampling frequency has the period which is long (frequency which is small) to such an extent that no influence is exerted by the variation of the center of gravity caused by any other factor than the large body motion such as the small body motion, the respiration or the like.

In Fig. 5(b), the locus between the point A1 and the point B1 moves rightward at a speed beyond a predetermined value, for example. Therefore, the large body motion information estimating unit 621 specifies the locus in that section as the large body motion locus. In the same manner, the locus between the point B2 and the point C1 moves leftward at a speed beyond the predetermined value, for example. Therefore, the large body motion information estimating unit 621 specifies the locus in that section as the large body motion locus, too.

Next, the large body motion information estimating unit 621 estimates the contents of the large body motion of the subject S on the basis of the specified large body motion locus. In particular, for example, if the large body motion locus is a linear locus along a widthwise direction of the bed BD, then it is estimated that the subject S has turned over. As another example, if the large body motion locus is a linear locus along a lengthwise direction of the bed BD, then it is estimated that the subject S has raised (gotten up) his/her upper body.

As other examples, it is possible to let the storage unit 7 store a table created for showing a relationship between the large body motions of the subject S in various aspects, and the large body motion loci caused due to those large body motions. Then, the large body motion information estimating unit 621 may estimate the appearance of the large body motion of the subject S by comparing the specified large body motion locus with the table stored in the storage unit 7.

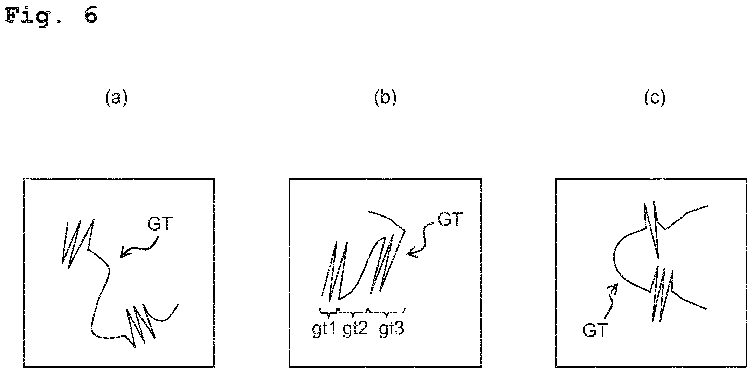

The large body motion information estimating unit 621 estimates the contents of the large body motion of the subject S and, at the same time as or before or after that, removes the large body motion locus(loci) from the center of gravity locus GT so as to send the center of gravity locus GT, where the large body motion locus(loci) is(are) removed, to the small body motion information estimating unit 622. Figs. 6(a) to 6(c) depict loci obtained by removing the large body motion loci from the center of gravity locus GT depicted in Fig. 5(a). Fig. 6(a) depicts the center of gravity locus GT in the area A of Fig. 5(a), Fig. 6(b) depicts the center of gravity locus GT in the area B of Fig. 5(a), and Fig. 6(c) depicts the center of gravity locus GT in the area C of Fig. 5(a). Each of those loci corresponds to the center of gravity locus GT in the stable posture period.

Next, the body motion information estimating unit 62 uses the small body motion information estimating unit 622 to separate the center of gravity locus GT from which the large body motion locus has (loci have) been removed, into the small body motion locus and the respiratory oscillation locus so as to specify the small body motion locus. A particular process will be explained with an example of separating the center of gravity locus GT of the area B (Fig. 6(b)) into the small body motion locus and the respiratory oscillation locus.

The small body motion information estimating unit 622 can analyze the position of center of gravity G at each sampling time, and properly separate the locus of the movement of the center of gravity G according to a small body motion (the small body motion locus) from the respiratory oscillation locus, on the basis of the definition of the "small body motion" and/or the feature of the respiratory oscillation locus (that is, the feature of periodical oscillation along the body axis direction). In particular, for example, a center of gravity locus which is included in the center of gravity locus GT and which periodically oscillates in a specific direction (in the body axis direction) is regarded as the respiratory oscillation locus, and a center of gravity locus different from that kind of center of gravity locus is regarded as the small body motion locus.

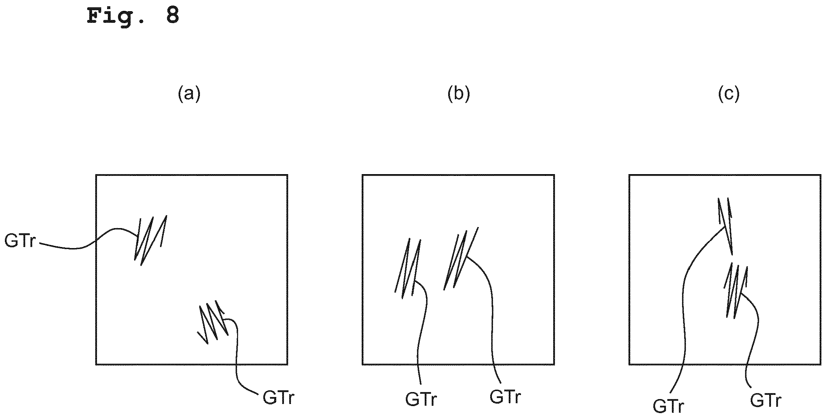

In Fig. 6(b), the center of gravity locus GT includes parts gt1 and gt3 exhibiting the movement of the center of gravity G due only to the respiration, and a part gt2 exhibiting the movement of the center of gravity G due to the respiration and the small body motion. The part gt2 exhibiting the movement of the center of gravity G due to the respiration and the small body motion is different from the parts gt1 and gt3 exhibiting the movement of the center of gravity G due only to the respiration in that the center of gravity locus of the part gt2 does not oscillate periodically in the specific direction (note that the oscillation loci of the parts gt1 and gt3 are present repetitively on one axis along the oscillation direction in reality but in Fig. 6(b), for the sake of explanation, they are drawn with such a deviation as along the direction orthogonal to the body axis direction. Much the same is true on Figs. 8(a) to 8(c) and Figs. 9 and 11).

Therefore, as one method for separating and extracting the small body motion locus, only the center of gravity loci (gt1, gt3) oscillating periodically in the specific direction are regarded as the respiratory oscillation loci and then removed, whereas the other part (gt2) is separated and extracted as the small body motion locus. The separation and the extraction as described above, for example, can be carried out in accordance with the method as described below.

The center of gravity variation, which is repeated periodically and which is included in the center of gravity variations provided during a stable respiration period (that is, a period in which the subject S perform only respiration, without any body movement) in the past, is detected by means of the frequency analysis such as the Fourier analysis or the like. The direction of the center of gravity change exhibited in the corresponding frequency is detected, and this is regarded as the center of gravity variation caused by the respiration. After that, the difference between the presently measured center of gravity variation and the respiratory oscillation locus is extracted as the small body motion locus.

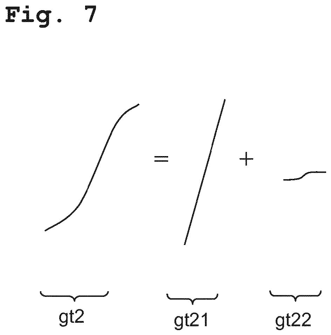

Another method is available as depicted in Fig. 7. That is, the portion (gt2), which does not form the center of gravity locus oscillating periodically in any specified direction, is decomposed into the portion gt21 which constitutes a part of the center of gravity locus oscillating periodically in a specified direction and the other portion gt22. Then, only the other part gt22 is regarded as the small body motion locus, and is separated and extracted.

The small body motion information estimating unit 622 determines the small body motion of the subject on the basis of the specified small body motion locus. In particular, for example, based on the length of the small body motion locus (the movement amount of the center of gravity G), the small body motion information estimating unit 622 estimates whether the arms of the subject S has moved or the legs of the subject S has moved. Generally, the small body motion locus shown (caused) by a heavier leg's movement is longer than the small body motion locus shown (caused) by an arm's movement. As another example, by specifying characteristic small body motion locus caused due to the head's turning movement, the small body motion information estimating unit 622 estimates that the orientation of the head of the subject S has changed.

As other examples, it is possible to let the storage unit 7 store a table created for showing a relationship between the small body motions of the subject S in various aspects, and the small body motion loci caused due to those small body motions. Then, the small body motion information estimating unit 622 may estimate the appearance of the small body motion of the subject S by comparing the specified small body motion locus with the table stored in the storage unit 7.

The small body motion information calculating unit 622 estimates the contents of the small body motion information and, at the same time as or before or after that, sends the respiratory oscillation locus obtained by separating the small body motion locus (loci) from the center of gravity variation, to the respiration information calculating unit 63.

The respiratory oscillation loci GTr, which are extracted from the center of gravity loci GT depicted in Figs. 6(a) to 6(c), are depicted in Figs. 8(a) to 8(c) respectively. The number of times of the reciprocating motion of the respiratory oscillation loci GTr depicted in Figs. 8(a) to 8(c) represents the respiratory rate of the subject S. Therefore, the respiration information calculating unit 63 calculates the respiratory rate (such as the respiratory rate per one minute) of the subject S on the basis of the respiratory oscillation loci GTr depicted in Figs. 8(a) to 8(c).

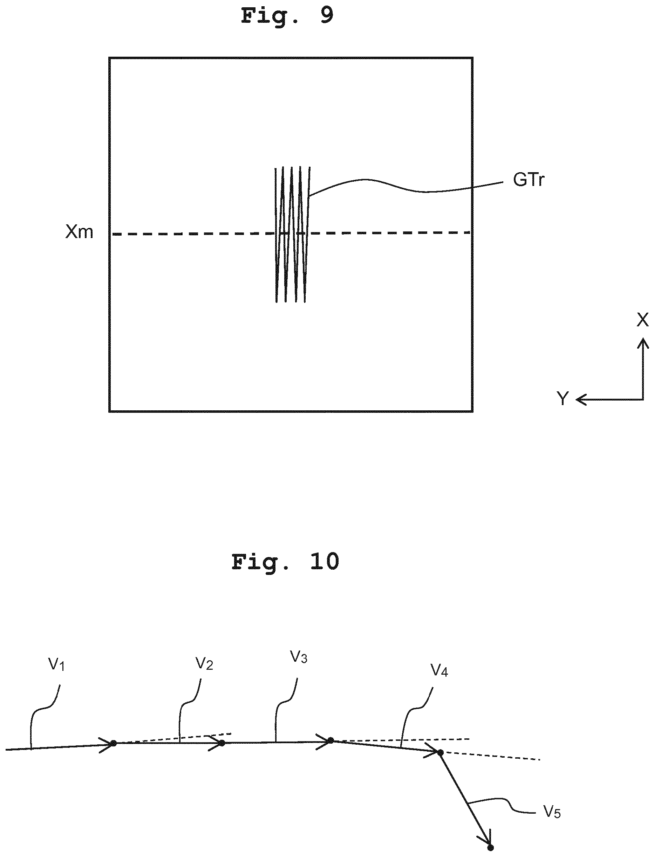

Specifically, the respiration information calculating unit 63 firstly rotates the respiratory oscillation loci GTr of the center of gravity locus GT of the subject S so that the oscillation direction of the respiratory oscillation loci GTr is coincident with the X axis direction (Fig. 9). Subsequently, the respiration information calculating unit 63 performs the filtering of a plurality of stages for the respiratory oscillation loci GTr depicted in Fig. 9 by using a multi-stage filter bank. The high frequency component is removed as the noise in the filtering at each stage. On the other hand, the filtering at the next stage is performed for the low frequency component obtained by the filtering at each stage. After performing the filtering a number of times corresponding to the predetermined number of stages, the low frequency component obtained at the final stage can be regarded as the number of times of respiration.

Next, the respiration information calculating unit 63 estimates the ventilation volume of one respiration cycle of the subject S, on the basis of the respiratory oscillation locus GTr (Fig. 9). Note that the respiratory ventilation volume is the physical amount corresponding to the depth of respiration.

In the case of the large and deep respiration, when the lungs expand during the inhalation, then the diaphragm is greatly moved and lowered downwardly as compared with the ordinary inhalation, and the internal organs are also greatly moved downwardly. On the other hand, upon the expiration, i.e., when the lungs shrink, then the diaphragm is greatly moved and raised upwardly as compared with the ordinary expiration, and the internal organs are also greatly moved upwardly. On the contrary, in the case of the small and shallow respiration, the movement of the internal organs is small as compared with the ordinary state. According to the research performed by the inventors of the present invention, it has been found out that the slight movement of the center of gravity G caused by the movement of internal organs is affected by the size or magnitude of the respiration. Specifically, the amplitude is increased as compared with the ordinary state when the respiration is large and deep, while the amplitude is decreased as compared with the ordinary state when the respiration is small and shallow. The ventilation volume of one respiration cycle can be calculated by being correlated with the amplitude. For example, the following procedure is performed in advance. That is, the subject performs the large and deep respiration in a state in which the subject lies on his/her back on the bed, and the ventilation volume and the amplitude obtained in this state are recorded beforehand. Further, the subject performs the small and shallow respiration, and the ventilation volume and the amplitude obtained in this state are recorded beforehand. The respiratory ventilation volume is calculated using the amplitude of the acquired center of gravity locus based on the respiration. It is also possible to calculate a minute volume (a ventilation volume per one minute) by calculating the ventilation volume of one respiration cycle. When the number of times of respiration per one minute and the minute volume are known, it is thereby possible to monitor whether the respiratory condition of the subject S is comprehensively in a good state or in a bad state.

<The image information detection step S5, the audio information detection step S6, and the temperature information detection step S7>

In the image information detection step S5, the image information detecting unit 3 shoots the bed BD, detects image information about the subject S, and sends the detected image information to the control unit 6. The image information detected by the image information detecting unit 3 is, for example, an appearance (aspect) of the body motion of the subject on the bed BD. Further, an image of the upper surface of the bed BD absent of the subject S is also a kind of image information and, for example, may be used for determining whether or not the subject S is present on the bed.

In the audio information detection step S6, the audio information detecting unit 4 detects audio information about the subject S on the bed BD, and sends the detected audio information to the control unit 6. The audio information detected by the audio information detecting unit 4 is, for example, the utterance, somniloquy, respiratory sound, snore, sneeze, yawn and the like of the subject S.

In the temperature information detection step S7, the temperature (thermal) information detecting unit 5 detects temperature (thermal) information about the subject S on the bed BD, and sends the detected temperature information to the control unit 6. The temperature information detected by the temperature information detecting unit 5 is, for example, temperature distribution of the body surface of the subject S.

The control unit 6 synchronizes following items of information and sends the same to the condition determining unit 64. The items are the center of gravity locus GT of the subject S calculated in the center of gravity position calculation step S2, the body motion information of the subject S estimated in the body motion information estimation step S3, the respiration information of the subject S calculated in the respiration information calculation step S4, the image information of the subject S detected in the image information detection step S5, the audio information of the subject S detected in the audio information detection step, and the temperature information of the subject S detected in the temperature information detection step.

<The condition determination step S8>

In the condition determination step S8, the condition determining unit 64 determines various conditions of the subject S on the basis of at least one item of the center of gravity position (the center of gravity locus), body motion information and respiration information of the subject S, and at least one item of the image information, audio information and temperature information of the subject S. The following items are an example of the conditions of the subject S determined by the condition determining unit 64.

(1) Contents of the small body motion;
(2) Respiratory condition;
(3) Dangerous condition of falling
(4) Asleep/awake;
(5) Time of deathwatch; and
(6) Determination of life/death

(1) Contents of the small body motion

The condition determining unit 64 can determine contents of the small body motion of the subject S, based on the contents of the small body motion of the subject S estimated by the body motion information estimating unit 62, and on the appearance of the body motion of the subject S detected by the image information detecting unit 3.

Different from the large body motion, the small body motion includes comparatively complicated movements of the hands and feet, and thus a certain shape of small center of gravity locus may correspond to a plurality of types of the small body motion. For example, because the right arm and the left arm are usually almost the same in weight, there is almost equivalence between a small center of gravity locus shown when the left arm moves rightward through a predetermined distance, and a small center of gravity locus shown when the right arm moves rightward through the identical predetermined distance.

If the small body motion information estimating unit 622 cannot estimate the contents of the small body motion based on the small center of gravity locus, then the condition determining unit 64 uses the appearance of the body motion of the subject S detected by the image information detecting unit 3, to determine the contents of the small body motion of the subject S.

In this manner, it is possible to precisely determine the contents of the small body motion of the subject S by using the image information fed from the image information detecting unit 3 as necessary.

Note that the image information detecting unit 3 does not need to constantly shoot for determining the contents of the small body motion but, for example, may perform shooting only when it is conceivable that the center of gravity G of the subject S moves in a direction different from the body axis direction, and a body motion different from the respiration is caused. Further, the image information about the subject S detected by the image information detecting unit 3 may be deleted at the point of confirming that there will not be any use thereof for determining the small body motion. In this manner, it is possible to protect the privacy of the subject S in sleep by keeping the images as few as possible in shooting and storage.

Further, because it is possible to know the posture of the subject S on the bed BD with the temperature information (the temperature distribution of the body surface) from the temperature information detecting unit 5, instead of the image information fed from the image information detecting unit 3, the temperature information fed from the temperature information detecting unit 5 may be used. It is also possible to protect the privacy of the subject S by knowing the posture of the subject S based on the temperature distribution of the body surface without shooting the face and/or facial expression of the subject S.

Note that in the same manner as in determining the contents of the small body motion, it is also possible to precisely determine the contents of the large body motion of the subject S, based on the contents of the large body motion of the subject S estimated by the body motion information estimating unit 62, and on the appearance of the body motion of the subject S detected by the image information detecting unit 3.

(2) Respiratory condition

The condition determining unit 64 can determine the respiratory condition of the subject S, based on the respiration information of the subject S calculated by the respiration information calculating unit 63, and the audio information (respiratory sound) of the subject S detected by the audio information detecting unit 4.

The respiratory condition determined by the condition determining unit 64 is, as an example, utterance, snore, somniloquy, obstructive apnea, and the like.

When the subject S performs an utterance or somniloquy or snore, the utterance, snore or somniloquy appears as tiny noises in the respiratory oscillation locus GTr sent from the body motion information estimating unit 62 to the respiratory rate calculating unit 63. Therefore, for example, if the condition determining unit 64 receives a notice from the respiration information calculating unit 63 indicating that noises are occurring in the respiratory oscillation locus GTr, then the condition determining unit 64 refers to the respiratory sound of the subject S detected by the audio information detecting unit 4 to determine whether the noises are caused by the utterance, snore or somniloquy.

Further, if the subject S has come into the condition of obstructive apnea which is the main symptom of sleep apnea syndrome, the upper airway of the subject S is obstructed so that the respiration is suppressed, and therefore, the amplitude of the respiratory oscillation locus GTr becomes extremely small. Then, along with the restart of the respiration of the subject S, the amplitude of the respiratory oscillation locus GTr will become temporarily large. Therefore, for example, if the condition determining unit 64 receives a notice from the respiration information calculating unit 63 indicating that the amplitude of the respiratory oscillation locus GTr is not more than a predetermined value, then the condition determining unit 64 refers to the respiratory sound of the subject S detected by the audio information detecting unit 4 to determine whether or not the subject S stays in the condition of obstructive apnea.

In this manner, it is possible to more precisely determine the respiratory condition of the subject S by the determination using not only the respiratory oscillation locus GTr but also the audio information of the subject S detected by the audio information detecting unit 4.

Note that if the noises included in the respiratory oscillation locus are determined as caused by any of the utterance, snore and somniloquy, then the respiration information calculating unit 63 may first remove those noises and then calculate the respiratory rate and the respiratory ventilation volume. By performing such noise cancelling, it is possible to more precisely calculate the respiratory rate and the respiratory ventilation volume.

(3) Dangerous condition of falling

The condition determining unit 64 can determine whether or not the subject S is facing the dangerous condition of falling (that is, a condition in which a danger or risk of the subject S falling from the bed exists), based on the position of the center of gravity G of the subject S calculated by the center of gravity position calculating unit 61, and the image information of the subject S detected by the image information detecting unit 3.

As an example, if the distance between the center of gravity G of the subject S and an edge of the bed BD becomes not larger than a predetermined value, then the condition determining unit 64 refers to the image information of the subject S detected by the image information detecting unit 3. If the subject S is in a predetermined condition in the image information (such as one of the feet or hands is hanging off of the bed or the like), the condition determining unit 64 determines that the subject S is facing the dangerous condition of falling.

In this manner, it is possible to precisely determine whether or not the subject S is facing the dangerous condition of falling by the determination using not only the position of the center of gravity G of the subject S but also the image information of the subject S detected by the image information detecting unit 3. Note that it is possible to protect the privacy of the subject S, if the image information detecting unit 3 is configured to detect the image information if and only if the distance between the center of gravity G of the subject S and an edge of the bed BD becomes not longer than the predetermined value, or if the temperature distribution of the body surface detected by the temperature information detecting unit 5 is used instead of the image information.

(4) Asleep/awake

The condition determining unit 64 can determine whether the subject S is asleep or awake on the basis of the body motion of the subject S estimated by the body motion information estimating unit 62, the respiration information of the subject S calculated by the respiration information calculating unit 63, the temperature information of the subject S detected by the temperature information detecting unit 5, and the like.

As an example, the condition determining unit 64 determines that the subject S is asleep when it is observed that each of the frequency of the small body motion and the respiratory rate per one minute of the subject S being not more than a predetermined value, and the body temperature of the subject S (calculated on the basis of the temperature distribution of the body surface) decreasing beyond a predetermined range.

(5) Time of deathwatch

The condition determining unit 64 can determine whether or not the subject S is facing the time of deathwatch (that is, nearing death) on the basis of the body motion of the subject S estimated by the body motion information estimating unit 62, the respiration information of the subject S calculated by the respiration information calculating unit 63, the temperature information of the subject S detected by the temperature information detecting unit 5, and the like.

As an example, the condition determining unit 64 determines that the subject S is in the condition of facing deathwatch on the basis of the subject S decreasing in body motion, decreasing in body temperature, decreasing in the amplitude of respiratory oscillation locus (decreasing in the respiratory ventilation volume), decreasing in body weight (found by a detected value from the load detecting unit 1), and the like.

(6) Determination of life/death

The condition determining unit 64 determines whether the subject S is alive or dead by comprehensively using the body motion information of the subject S estimated by the body motion information estimating unit 62, the respiration information of the subject S calculated by the respiration information calculating unit 63, the image information of the subject S detected by the image information detecting unit 3, the audio information of the subject S detected by the audio information detecting unit 4, and the temperature information of the subject S detected by the temperature information detecting unit 5.

In particular, for example, the condition determining unit 64 can determine that the subject S is dead if the body motion and the respiration of the subject S have stopped under a certain condition, and the body temperature of the subject S has decreased to be not higher than a predetermined temperature. The certain condition can be set and determined by the user who is a doctor or the like.

<Display Step>

In the display step S9, a monitor displays the condition of the subject S determined by the condition determining unit 64. Further, the monitor also displays the respiratory rate and the respiratory ventilation volume calculated by the respiration information calculating unit 63. The user can visually watch the monitor to observe the respiratory rate, the respiratory ventilation volume, and various other conditions of the subject S.

The user of the bed monitoring system 100 can also set the system to cause the notifying unit 9 to notify the user if the subject S comes into a predetermined condition. For example, it is possible for the user to set the system, by using the input unit 10, such that the system performs the notification when the subject S is in the dangerous condition of falling or the condition of apnea, and/or the subject S is facing the time of deathwatch.

The effects of the bed monitoring system 100 of this embodiment are summarized as follows.

The bed monitoring system 100 of this embodiment determines the condition of the subject S, based on at least one item of the center of gravity position, body motion information and respiration information of the subject S obtained (found) by using the load detectors 11 to 14 arranged under the legs of the bed BD, and on at least one item of the image information, audio information and temperature information detected by the image information detecting unit 3, the audio information detecting unit 4 and the temperature information detecting unit 5. In this manner, because the condition is determined on the basis of various kinds of information about the subject, it is possible to precisely monitor various items about the subject.

Further, because each of the load detectors 11 to 14, the image information detecting unit 3, the audio information detecting unit 4, and the temperature information detecting unit 5 detects the information about the subject noninvasively, neither discomfort nor sense of incongruity is given to the subject.

The bed monitoring system 100 of this embodiment calculates the respiratory rate of the subject S by removing the locus of the movement of the center of gravity G caused by the body motion of the subject S from the center of gravity locus GT of the subject S, and extracting only the locus of the movement of the center of gravity G caused by the respiration of the subject S. Therefore, the degree of precision of the calculated respiratory rate is high.

It is also possible to adopt the following modified embodiments for the bed monitoring system 100 of the above embodiment and for monitoring the subject S by using the same.

The large body motion information estimating unit 621 of the body motion information estimating unit 62 of the bed monitoring system 100 of the above embodiment can also specify the large body motion locus included in the center of gravity locus GT of the subject S by the following method.

The large body motion information estimating unit 621 determines that the large body motion occurs if the center of gravity G of the subject S moves in almost one direction beyond a predetermined distance within a predetermined time, and specifies that the center of gravity locus GT during that period is the large body motion locus. As an example, it is possible to determine whether or not the center of gravity G moves in almost one direction on the basis of whether or not the angle, which is formed between the motion vector of the center of gravity G provided during a predetermined sampling period and the motion vector of the center of gravity G provided during the next sampling period, is not more than a predetermined angle.

Specifically, for example, as depicted in Fig. 10, each of the motion vectors $v_2$ to $v_4$ of the center of gravity G has the angle of not more than about 5° with respect to the motion vector during the immediately preceding sampling period. However, the motion vector $v_5$ has the angle of not less than 5° with respect to the motion vector $v_4$ during the immediately preceding sampling period. In such a situation, the following assumption can be made. That is, the center of gravity G moves in a substantially constant direction during the sampling period corresponding to each of the motion vectors $v_1$ to $v_4$, and the movement direction is changed during the sampling period corresponding to the motion vector $v_5$.

If the movement direction is regarded as changed, then the large body motion information estimating unit 621 determines whether or not the movement has occurred beyond the predetermined distance within the predetermined time, based on the motion vectors (the motion vectors $v_1$ to $v_4$ in this case) before the point of time when the movement direction is changed. Then, if the movement has occurred beyond the predetermined distance within the predetermined time, then the locus exhibited by those motion vectors is specified as the large body motion locus.

Note that the center of gravity locus GT may be subjected to the filtering with a low-pass filter prior to specify the large body motion locus based on the use of the motion vector. Accordingly, the high frequency component (noise) is removed, and it is possible to improve the accuracy of specifying.

The small body motion information estimating unit 622 of the body motion information estimating unit 62 of the bed monitoring system 100 of the above embodiment can also specify the small body motion locus and respiratory oscillation locus of the subject S, in accordance with the method of outlier removal.

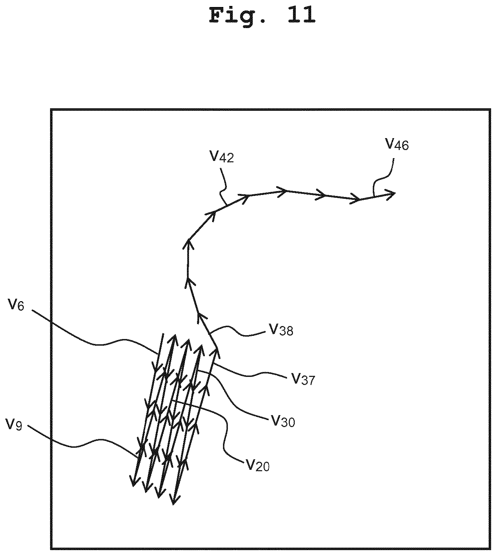

Specifically, it is assumed that the center of gravity locus from which the large body motion locus has been removed includes 41 motion vectors from $v_6$ to $v_{46}$ as depicted in Fig. 11. At first, the small body motion information estimating unit 622 acquires the direction of the mode vector $v_f$ from the 41 motion vectors. The motion vectors $v_6$ to $v_{46}$ have the directions respectively. However, as depicted in Fig. 11, some of the motion vectors $v_6$ to $v_{46}$ have mutually identical directions respectively. The direction of the mode vector $v_f$ is equal to the direction which appears most frequently and which is included in the directions of the motion vectors $v_6$ to $v_{46}$. As clarified from Fig. 10, the direction of the mode vector $v_f$ is equal to the direction of any one of the motion vectors $v_6$ to $v_{37}$.

Subsequently, the small body motion information estimating unit 622 regards, as the majority vector, such a motion vector included in the motion vectors $v_6$ to $v_{46}$ that the difference between the direction of the vector itself and the direction of the mode vector $v_f$ (or the direction having an angle of 180° with respect to the direction of the mode vector $v_f$) is not more than a certain threshold value, while the small body motion information estimating unit 622 regards, as the minority vector, such a motion vector that the difference between the direction of the vector itself and the direction of the mode vector $v_f$ (and the direction having an angle of 180° with respect to the direction of the mode vector $v_f$) is larger than a certain threshold value. Specifically, the motion vectors $v_6$ to $v_{37}$, which have the direction extending substantially along the body axis direction of the subject S, are regarded as the majority vectors, and the other motion vectors $v_{38}$ to $v_{46}$ are regarded as the minority vectors. Then, the majority vectors are removed (minority vectors are extracted). By doing so, the small body motion information estimating unit 622 specifies the respiratory oscillation locus included in the center of gravity locus GT where the large body motion locus is already removed and, furthermore, specifies the small body motion locus.

The control unit 3 of the bed monitoring system 100 may be further built therein with a heart rate calculating unit. The heart rate calculating unit extracts the heart beat component from the load signal fed from the load detecting unit 1. Specifically, the following method is used. The heart beat component is the signal component existing in a band of 0.5 Hz to 2.5 Hz. Therefore, the heart rate calculating unit extracts the signal components in this frequency band from the output values of the four load detectors 11 to 14. Subsequently, the heart rate calculating unit calculates the center of gravity locus based on the heart beat component in accordance with the same or equivalent method as that used in the center of gravity locus calculating step S2.

The inventors have found out that the center of gravity locus based on the heart beat component is oscillating (vibrating) in a direction inclined to the body axis direction according to the heart beat. Therefore, it is possible to specify the heart rate by specifying the number of times of the oscillation (vibration).

Further, the heart beat information calculating unit estimates the cardiac output of one heart beat cycle of the subject S, on the basis of the center of gravity locus based on the heart beat. The amplitude of one cycle corresponds to one heart beat cycle. Therefore, the cardiac output of one heart beat cycle can be estimated by being correlated with the amplitude. For example, the following procedure is performed in advance. That is, the subject is in a state in which the subject lies on his/her back on the bed, and the cardiac output and the amplitude obtained in this state are recorded beforehand. The cardiac output is calculated from the amplitude on the basis of the acquired center of gravity locus based on the heart beat. According to the heart rate and the cardiac output, it is possible to monitor whether the blood pressure state of the subject S is comprehensively in a good state or in a bad state. Further, it is possible to monitor whether the health status (condition of health) of the subject S is comprehensively in a good state or in a bad state by using, in the monitoring, the number of times of respiration per one minute and the ventilation volume per one minute described above as well.

The condition determining unit 64 can refer to the heart rate and the cardiac output, in the condition determination step S8, to determine the asleep/awake condition, to determine the time of deathwatch, to determine the life/death condition, and the like.

In the bed monitoring system 100 of the above embodiment, the audio information detecting unit 4 may include slim microphone provided on the upper surface of a bed board BD2 so as to locate under the bedclothes (bedding), by replacing or in addition to the slim microphone provided on the head board BD1 of the bed BD. With such kind of microphone(s), it is possible to detect the cardiac sound of the subject S as the audio information of the subject S.

The cardiac sound detected by the audio information detecting unit 4 can be used for calculating the heart rate, for example. Further, the user, who is a doctor, can perform a simplified medical examination somewhere away from the bed, by confirming the cardiac sound of the subject S while monitoring the appearance of the body motion and respiration of the subject S displayed on the display unit 8 of the bed monitoring system 100.

The bed monitoring system 100 of the above embodiment does not need to include all of the image information detecting unit 3, the audio information detecting unit 4 and the temperature information detecting unit 5, but may have only one of those units. In the present invention and this specification, the term "subject information detecting unit" is used to refer collectively to at least one of the image information detecting unit, the audio information detecting unit and the temperature information detecting unit included in the bed monitoring system.

In the bed monitoring system 100 of the embodiment described above, the respiration information calculating unit 63 calculates the respiratory rate of the subject S by using the wavelet transformation. However, it is also possible to use other methods. Specifically, for example, the point positioned on the most positive side in the X axis direction and the point positioned on the most negative side in the X axis direction are firstly found from the respiratory oscillation locus GTr depicted in Fig. 9, so as to calculate the intermediate value Xm of the X coordinates of the both points. As depicted in Fig. 9, the intermediate value Xm can be regarded as the center of oscillation of the respiratory oscillation locus GTr. Subsequently, the respiration information calculating unit 63 finds the number of times of movement of the respiratory oscillation locus GTr from the negative side to the positive side (or the positive side to the negative side) in the X axis direction while crossing over the intermediate value Xm. Based on the found number of times, the respiration information calculating unit 63 calculates the frequency of respiratory oscillation locus GTr, i.e., the respiratory rate.

In the embodiment described above, each of the load detectors 11, 12, 13, 14 is not limited to a load sensor having a beam-type load cell. It is also possible to use, for example, a force sensor.

Note that in the embodiment described above, the number of load detectors is not limited to four. It is also allowable to use five or more load detectors by providing an additional leg or additional legs for the bed BD. Alternatively, it is also allowable to arrange the load detectors for only three of the legs of the bed BD. Even when the three load detectors are used, it is possible to detect a position of the center of gravity G of the subject S on the plane of the bed BD provided that the three load detectors are not arranged on a straight line.

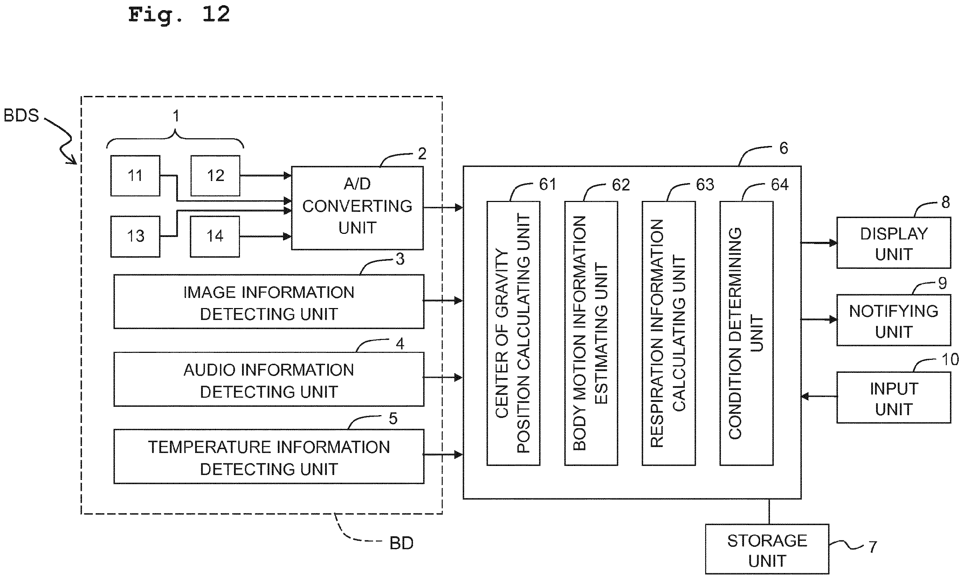

In the embodiment described above, the load detectors 11, 12, 13, 14 are arranged respectively under the casters $C_1$, $C_2$, $C_3$, $C_4$ attached to the lower ends of the legs of the bed BD. However, there is no limitation thereto. Each of the load detectors 11, 12, 13, 14 may be provided respectively between one of the four legs of the bed BD and the board (bed board) of the bed BD. Alternatively, if each of the four legs of the bed BD can be divided into upper and lower portions, each of the load detectors 11, 12, 13, 14 may be provided between upper leg and lower leg. Further alternatively, the load detectors 11, 12, 13, 14 may be formed integrally with the bed BD to construct a bed system BDS comprising the bed BD and the bed monitoring system 100 of this embodiment (Fig. 12). Note that in this specification, the "load detector placed in the bed" mean the load detector which is provided between one of the four legs of the bed BD and the board of the bed BD, and the load detector which is provided between the upper leg and the lower leg, as described above.

Note that in the embodiment described above, it is also allowable to provide a signal amplifying unit for amplifying the load signal fed from the load detecting unit 1 and/or a filtering unit for removing the noise from the load signal, between the load detecting unit 1 and the A/D converting unit 2.

Note that in the bed monitoring system 100 of the embodiment described above, the display unit 8 is not limited to a unit which displays the information on the monitor so that the user can make the visual recognition. For example, the display unit 8 may be a printer which periodically prints and outputs the condition of the subject S. Alternatively, the display unit 8 may be a unit which performs the display by using a simple visual expression such that a blue lamp is turned ON in the case of the asleep state, a yellow lamp is turned ON in the case of the awake state, and/or a red lamp is turned ON in the apnea state. Further alternatively, the display unit 5 may be a unit which reports the condition of the subject S to the user by means of any sound or voice. Further alternatively, it is also allowable that the bed monitoring system 100 does not have the display unit 8 at all. The bed monitoring system 100 may have only an output terminal for outputting the information. A monitor (display device) or the like, which is provided to perform the display, will be connected to the bed monitoring system 100 by the aid of the output terminal.

Note that the notifying unit 9 of the embodiment described above performs the notification auditorily. However, the notifying unit 9 may be constructed to perform the notification visually by means of, for example, the flashing or flickering of light. Alternatively, the notifying unit 9 may be constructed to perform the notification by means of the vibration. Further, it is also allowable that the bed monitoring system 100 of the embodiment described above does not have the notifying unit 9 at all.

Note that the components of the bed monitoring system 100 of the embodiment described above, which are connected to one another by means of the wirings, may be connected to one another in a wireless manner.

The present invention is not limited to the embodiment described above provided that the feature of the present invention is maintained. Other embodiments, which are conceivable within the scope of the technical concept of the present invention, are also included in the scope of the present invention, where the scope of the invention is defined by the claims.

INDUSTRIAL APPLICABILITY

According to the bed monitoring system of the present invention, it is possible to precisely and noninvasively determine the condition of the subject on the bed from various points of view. Therefore, if the bed monitoring system of the present invention is used in the sites of medical practice and care service, then it is possible to give preferable care to the patients and care receivers while lessening the burden on the medical personnel and the care givers.

PARTS LIST

1: load detecting unit, 11, 12, 13, 14: load detector, 2: A/D converting unit, 3: image information detecting unit, 4: audio information detecting unit, 5: temperature information detecting unit, 6: control unit, 61: center of gravity position calculating unit, 62: body motion information estimating unit, 63: respiration information calculating unit, 64: condition determining unit, 7: storage unit, 8: display unit, 9: notifying unit, 10: input unit, 100: bed monitoring system, BD: bed, BDS: bed system, GT: center of gravity locus, S: subject.

**Claims**

1. A bed monitoring system (100) for monitoring a subject on a bed, the system (100) comprising:

a plurality of load detectors (11-14) which are to be placed in the bed or under legs of the bed and which are configured to detect a load of the subject;
a center (61) of gravity position calculating unit configured to obtain a temporal variation of a center of gravity position of the subject based on the detected load of the subject;
a body motion information determining unit (62) configured to obtain a body motion information based on the obtained temporal variation of the center of gravity position of the subject, the body motion information being an information on a movement of a whole or a part of the whole body of the subject different from a movement caused by a respiration of the subject;

a respiratory rate calculating unit (63) configured to calculate a respiratory rate of the subject based on the obtained temporal variation of the center of gravity position of the subject and the body motion information obtained by the body motion information determining unit;

**characterised in that** the system further comprises:

a subject information detecting unit configured to detect at least one of an image information of the subject, and a temperature information of the subject; and

a condition determining unit (64) configured to determine contents of a body motion of the subject by using both of the body motion information of the subject, and the image information of the subject or the temperature information of the subject,

wherein the condition determining unit (64) is configured to determine the contents of the body motion of the subject based only on the image information of the subject or the temperature information of the subject in a case that the contents of the body motion of the subject cannot be determined based on the body motion information of the subject.

2. The bed monitoring system (100) according to claim 1, wherein the body motion information includes an information on a large body motion of the subject and an information on a small body motion of the subject, an amount of movement of the center of gravity position of the subject within a predetermined time period caused by the small body motion being smaller than an amount of movement of the center of gravity position of the subject within the predetermined time period caused by the large body motion, and

the body motion information determining unit (62) includes a first body motion information determining unit (62) configured to determine the information on the large body motion of the subject and a second body motion information determining unit (62) configured to determine the information on the small body motion of the subject.

3. The bed monitoring system (100) according to claim 2, wherein the condition determining unit (64) is configured to determine contents of the small body motion of the subject based on the image information of the subject or the temperature information of the subject in a case that the contents of the small body motion of the subject cannot be determined based on the information on the small body motion of the subject.

4. The bed monitoring system (100) according to any one of claims 1 to 3, wherein the subject information detecting unit includes an audio information detecting unit (4) configured to detect an audio information of the subject.

5. A bed system (100) comprising:

a bed; and

the bed monitoring system (100) as defined in any one of claims 1-4.

**Patentansprüche**

1. Bettüberwachungssystem (100) zum Überwachen eines Subjekts auf einem Bett, wobei das System (100) umfasst:

eine Vielzahl von Lastdetektoren (11-14), die in dem Bett oder unter Beinen des Betts platziert werden sollen und die konfiguriert sind, um eine Last des Subjekts zu erfassen;

eine Mitte (61) der Schwerkraftpositionsberechnungseinheit, konfiguriert, um eine zeitliche Variation einer Schwerpunktposition des Subjekts basierend auf der erfassten Last des Subjekts zu erhalten;

eine Körperbewegungsinformationsbestimmungseinheit (62), konfiguriert, um Körperbewegungsinformationen basierend auf der erhaltenen zeitlichen Variation der Schwerpunktposition des Subjekts zu erhalten, wobei die Körperbewegungsinformationen Informationen über eine Bewegung eines gesamten oder eines Teils des gesamten Körpers des Subjekts sind, die sich von einer Bewegung unterscheidet, die durch eine Atmung des Subjekts verursacht wird;

eine Atemfrequenzberechnungseinheit (63), konfiguriert, um eine Atemfrequenz des Subjekts basierend auf der erhaltenen zeitlichen Variation der Schwerpunktposition des Subjekts und der Körperbewegungsinformationen, die durch die Körperbewegungsinformationsbestimmungseinheit erhalten werden, zu berechnen;

**dadurch gekennzeichnet, dass** das System ferner umfasst:

eine Subjektinformationserfassungseinheit, konfiguriert, um mindestens eines von Bildinformationen des Subjekts und Temperaturinformationen des Subjekts zu erfassen; und

eine Zustandsbestimmungseinheit (64), konfiguriert, um Inhalte einer Körperbewegung des Subjekts unter Verwendung sowohl der Körperbewegungsinformationen des Subjekts als auch der Bildinformationen des Subjekts oder der Temperaturinformationen des Subjekts zu bestimmen,

wobei die Zustandsbestimmungseinheit (64) konfiguriert ist, um die Inhalte der Körperbewegung des Subjekts nur basierend auf den Bildinformationen des Subjekts oder den Temperaturinformationen des Subjekts in einem Fall zu bestimmen, in dem die Inhalte der Körperbewegung des Subjekts nicht basierend auf den Körperbewegungsinformationen des Subjekts bestimmt werden kann.

2. Bettüberwachungssystem (100) nach Anspruch 1, wobei die Körperbewegungsinformationen Informationen über eine große Körperbewegung des Subjekts und Informationen über eine kleine Körperbewegung des Subjekts, ein Ausmaß an Bewegung der Schwerpunktposition des Subjekts innerhalb eines vorbestimmten Zeitraums, die durch die kleine Körperbewegung verursacht wird, die kleiner ist als ein Ausmaß an Bewegung der Schwerpunktposition des Subjekts innerhalb des vorbestimmten Zeitraums, die durch die große Körperbewegung verursacht wird, einschließen, und

die Körperbewegungsinformationsbestimmungseinheit (62) eine erste Körperbewegungsinformationsbestimmungseinheit (62), konfiguriert, um die Informationen über die große Körperbewegung des Subjekts zu bestimmen, und eine zweite Körperbewegungsinformationsbestimmungseinheit (62), konfiguriert, um die Informationen über die kleine Körperbewegung des Subjekts zu bestimmen, einschließt.

3. Bettüberwachungssystem (100) nach Anspruch 2, wobei die Zustandsbestimmungseinheit (64) konfiguriert ist, um Inhalte der kleinen Körperbewegung des Subjekts basierend auf den Bildinformationen des Subjekts oder den Temperaturinformationen des Subjekts in einem Fall zu bestimmen, in dem die Inhalte der kleinen Körperbewegung des Subjekts nicht basierend auf den Informationen über die kleine Körperbewegung des Subjekts bestimmt werden können.

4. Bettüberwachungssystem (100) nach einem der Ansprüche 1 bis 3, wobei die Subjektinformationserfassungseinheit eine

Audioinformationserfassungseinheit (4) einschließt, konfiguriert, um Audioinformationen des Subjekts zu erfassen.

5. Bettsystem (100), umfassend:

ein Bett; und
das Bettüberwachungssystem (100) wie definiert nach einem der Ansprüche 1-4.

**Revendications**

1. Système de surveillance de lit (100) pour la surveillance d'un sujet sur un lit, le système (100) comprenant :

une pluralité de détecteurs de charge (11-14) qui doivent être placés dans le lit ou sous les pieds du lit et qui sont configurés pour détecter une charge du sujet ;
une unité de calcul de position du centre (61) de gravité configurée pour obtenir une variation temporelle d'une position de centre de gravité du sujet sur la base de la charge détectée du sujet ;
une unité de détermination d'informations de mouvement corporel (62) configurée pour obtenir des informations de mouvement corporel sur la base de la variation temporelle obtenue de la position de centre de gravité du sujet, les informations de mouvement corporel étant des informations concernant un mouvement d'une entièreté ou d'une partie du corps entier du sujet différent d'un mouvement causé par une respiration du sujet ;
une unité de calcul de fréquence respiratoire (63) configurée pour calculer une fréquence respiratoire du sujet sur la base de la variation temporelle obtenue de la position de centre de gravité du sujet et des informations de mouvement corporel obtenues par l'unité de détermination d'informations de mouvement corporel ;
**caractérisé en ce que** le système comprend en outre :

une unité de détection d'informations de sujet configurée pour détecter au moins une parmi des informations d'image du sujet, et des informations de température du sujet ; et
une unité de détermination de condition (64) configurée pour déterminer un contenu d'un mouvement corporel du sujet en utilisant à la fois les informations de mouvement corporel du sujet et les informations d'image du sujet ou les informations de température du sujet,

dans lequel l'unité de détermination de condition (64) est configurée pour déterminer le contenu du mouvement corporel du sujet sur la base uniquement des informations d'image du sujet ou des informations de température du sujet dans un cas où le contenu du mouvement corporel du sujet ne peut pas être déterminé sur la base des informations de mouvement corporel du sujet.

2. Système de surveillance de lit (100) selon la revendication 1, dans lequel les informations de mouvement corporel incluent des informations concernant un grand mouvement corporel du sujet et des informations concernant un petit mouvement corporel du sujet, une quantité de mouvement de la position de centre de gravité du sujet au cours d'une période de temps prédéterminée causée par le petit mouvement corporel étant plus petite qu'une quantité de mouvement de la position de centre de gravité du sujet au cours de la période de temps prédéterminée causée par le grand mouvement corporel, et

l'unité de détermination d'informations de mouvement corporel (62) inclut une première unité de détermination d'informations de mouvement corporel (62) configurée pour déterminer les informations concernant le grand mouvement corporel du sujet et une deuxième unité de détermination d'informations de mouvement corporel (62) configurée pour déterminer les informations concernant le petit mouvement corporel du sujet.

3. Système de surveillance de lit (100) selon la revendication 2, dans lequel l'unité de détermination de condition (64) est configurée pour déterminer un contenu du petit mouvement corporel du sujet sur la base des informations d'image du sujet ou des informations de température du sujet dans un cas où le contenu du petit mouvement corporel du sujet ne peut pas être déterminé sur la base des informations concernant le petit mouvement corporel du sujet.

4. Système de surveillance de lit (100) selon l'une quelconque des revendications 1 à 3, dans lequel l'unité de détection d'informations de sujet inclut une unité de détection d'informations audio (4) configurée pour détecter des informations audio du sujet.

5. Système de lit (100) comprenant :

un lit ; et
le système de surveillance de lit (100) tel que défini dans l'une quelconque des revendications 1 à 4.

**Fig. 1**

100

1

11  12
13  14

2 — A/D CONVERTING UNIT

3 — IMAGE INFORMATION DETECTING UNIT

4 — AUDIO INFORMATION DETECTING UNIT

5 — TEMPERATURE INFORMATION DETECTING UNIT

6

61 — CENTER OF GRAVITY POSITION CALCULATING UNIT

62 — BODY MOTION INFORMATION ESTIMATING UNIT

63 — RESPIRATION INFORMATION CALCULATING UNIT

64 — CONDITION DETERMINING UNIT

8 — DISPLAY UNIT

9 — NOTIFYING UNIT

10 — INPUT UNIT

7 — STORAGE UNIT

EP 3 581 106 B1

Fig. 2

(a)

(b)

**Fig. 3**

```
┌─────────────────────────────────────────────┐
│          LOAD DETECTION STEP                  │──S1
└─────────────────────────────────────────────┘
                    │
                    ▼
┌─────────────────────────────────────────────┐
│   CENTER OF GRAVITY LOCUS CALCULATION STEP    │──S2
└─────────────────────────────────────────────┘
                    │
                    ▼
┌─────────────────────────────────────────────┐
│    BODY MOTION INFORMATION ESTIMATION STEP    │──S3
└─────────────────────────────────────────────┘
                    │
                    ▼
┌─────────────────────────────────────────────┐
│   RESPIRATION INFORMATION CALCULATION STEP    │──S4
└─────────────────────────────────────────────┘
                    │
                    ▼
┌─────────────────────────────────────────────┐
│      IMAGE INFORMATION DETECTION STEP         │──S5
└─────────────────────────────────────────────┘
                    │
                    ▼
┌─────────────────────────────────────────────┐
│      AUDIO INFORMATION DETECTION STEP         │──S6
└─────────────────────────────────────────────┘
                    │
                    ▼
┌─────────────────────────────────────────────┐
│   TEMPERATURE INFORMATION DETECTION STEP      │──S7
└─────────────────────────────────────────────┘
                    │
                    ▼
┌─────────────────────────────────────────────┐
│       CONDITION DETERMINATION STEP            │──S8
└─────────────────────────────────────────────┘
                    │
                    ▼
┌─────────────────────────────────────────────┐
│             DISPLAY STEP                      │──S9
└─────────────────────────────────────────────┘
```

**Fig. 4**

```
                                          ┌── 62
┌─────────────────────────────────────────────┐
│  ┌───────────────────────────────┐          │
│  │  CENTER OF GRAVITY LOCUS       │── 620    │
│  │  ACQUIRING UNIT                │          │
│  └───────────────────────────────┘          │
│  ┌───────────────────────────────┐          │
│  │  LARGE BODY MOTION             │── 621    │
│  │  INFORMATION ESTIMATING UNIT   │          │
│  └───────────────────────────────┘          │
│  ┌───────────────────────────────┐          │
│  │  SMALL BODY MOTION             │── 622    │
│  │  INFORMATION ESTIMATING UNIT   │          │
│  └───────────────────────────────┘          │
└─────────────────────────────────────────────┘
```

Fig. 5

(a)                                      (b)

EP 3 581 106 B1

Fig. 6

(a)                    (b)                   (c)

Fig. 7

## Fig. 8

(a)

(b)

(c)

## Fig. 9

## Fig. 10

Fig. 11

Fig. 12

CENTER OF GRAVITY POSITION CALCULATING UNIT 61
BODY MOTION INFORMATION ESTIMATING UNIT 62
RESPIRATION INFORMATION CALCULATING UNIT 63
CONDITION DETERMINING UNIT 64

DISPLAY UNIT 8
NOTIFYING UNIT 9
INPUT UNIT 10
STORAGE UNIT 7

A/D CONVERTING UNIT 2
IMAGE INFORMATION DETECTING UNIT 3
AUDIO INFORMATION DETECTING UNIT 4
TEMPERATURE INFORMATION DETECTING UNIT 5

11 12 13 14

BDS
BD

**EP 3 581 106 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011009085 A1
- JP 2008264338 A
- JP 4883380 B
- JP 4829020 B
- JP 4002905 B